# EUROPEAN PATENT SPECIFICATION

(11) **EP 2 321 401 B1**
(45) Date of publication and mention of the grant of the patent: **27.02.2019**
(21) Application number: 09784159.7
(22) Date of filing: 07.08.2009
(51) Int. Cl.: C12M 1/107, C12P 5/02

(54) **METHOD OF AND APPARATUS FOR FERMENTING BIOMASS**
VERFAHREN UND VORRICHTUNG ZUR VERGÄRUNG VON BIOMASSE
PROCÉDÉ ET APPAREIL DE FERMENTATION DE BIOMASSE

(30) Priority: 07.08.2008 FI 20080456
(43) Date of publication of application: 18.05.2011
(73) Proprietor: Toukonummi, Olavi, 25240 Hajala (FI)
(72) Inventor: Toukonummi, Olavi, 25240 Hajala (FI)
(74) Representative: Genip Oy
(86) International application number: PCT/FI2009/050651
(87) International publication number: WO 2010/015728

(56) References cited:
- EP-A- 0 033 017
- WO-A-2008/142007
- DE-A1-102006 008 026
- DE-C1- 19 805 580

## Description

### Technical field

The present invention relates to a method of and an apparatus for fermenting biomass in accordance with the preambles of the independent claims.

Thus, the invention especially relates to a method of fermenting organic material in an apparatus comprising a container, the method comprising the steps of: feeding organic material and fermenting microbes to the lower portion of the container, whereby material already existing in the container moves upwards as a plug flow, fermenting the upwards moving material and generating gas thereof, and removing the generated gas and completely fermented material from the upper portion of the container. Further, the invention especially relates to an apparatus for fermenting organic material, comprising a container, comprising a lower portion with mixing equipment and means for feeding organic material into the container, a fermenting zone above the lower portion, and an upper portion above the fermenting zone with means for removing gas and fermented material from the container.

### Background art

Biomass can be fermented by suitable microbes, in anaerobic conditions, to gas, liquid and solid material. Produced gas is in some applications nearly as good fuel as natural gas, and the liquid and solid materials produced in a fermenter are excellent plant fertilizers. When the fermentation is complete, the produced liquid and solid matter are almost odorless and sterile. Complete fermentation is aimed by using different methods in sealed containers. However, for reasons to be explained below, complete fermentation is only seldom achieved by using conventional fermentation apparatuses of today.

Uniform distribution of the fermenting microbes in the biomass to be fermented, as well as maintaining temperatures suitable for the process, is advantageous to the process. Therefore, the biomass to be fermented is advantageously both mixed and heated in the fermentation reactor. According to the known technology, fermentation process takes place in a sealed container or reactor. The reactor is equipped with suitable means for both heating up and mixing of the biomass. The biomass is kept in the container for a sufficient time for the fermentation to take place. Depending on conditions, complete fermentation can be achieved in 7 to 70 days.

The fermentation process can be either a wet process, whereby the solid material content is less than 15 %, or a dry process, whereby the solid material content is more than 15 %. Fermentation reactors can be either vertical reactors, in which the biomass to be fermented moves in vertical direction, or horizontal reactors, in which he biomass moves in horizontal direction.

DE-C1-198 05 580 discusses a continuously operating vertical fermentation reactor, i.e. a fermenter. The document is related to a problem that not yet fermented substrate leaves the container resulting in the decrease of gas yield. In the fermenter of the document the bottom part of the fermenter is divided into an annular outer inlet zone and an inner central discharge zone by means of an annular wall extending upwardly from the bottom of the fermenter. In operation fresh substrate is supplied tangentially into the annular inlet zone. Continuous feed of fresh substrate moves the annular contents of the zone upwards to the upper part of the fermenter where a mixer is arranged. The mixing together with the upwardly moving fresh substrate makes the substrate move to the center of the fermenter, where the substrate starts descending towards the bottom and the discharge outlet of the fermenter.

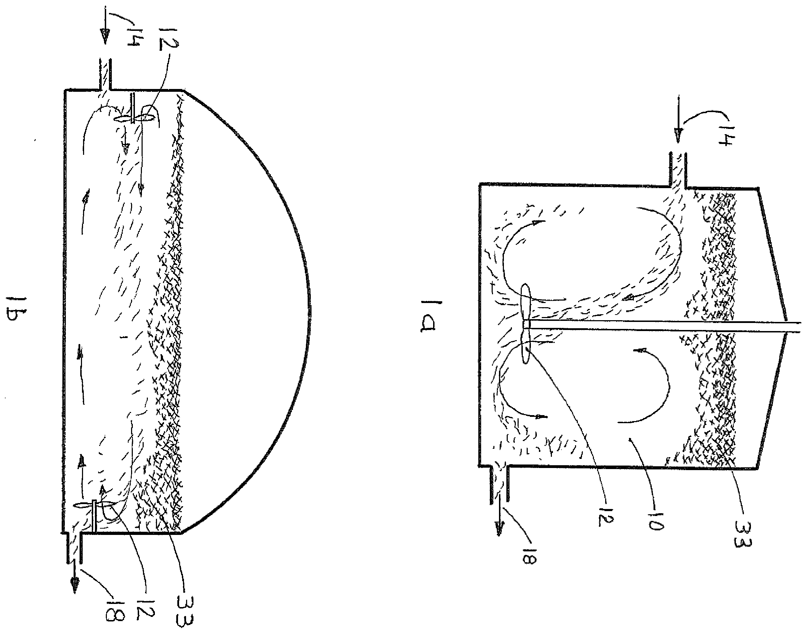

Both dry and wet processes can be used either in vertical or horizontal reactors. Figure 1a shows a vertical fermentation reactor, according to the state of the art, to be used in a wet process and Figure 1b shows a horizontal fermentation reactor, according to the state of the art, to be used in a wet process. In order to obtain sufficient mixing of the materials, the biomass to be fermented has to be fluent enough. Typical consistency, i.e. solid material content, to render complete mixing possible, is about 5 %.

Due to low solid material content and long fermentation time needed, a large volume of the fermentation reactor is required. In order to achieve a proper process temperature, a large volume of biomass, more than 95 % thereof being liquid, has to be heated. The large volume gives rise to high cost of the equipment. In addition to a large container volume, also the equipment for mixing and heating the biomass have to be large. Correspondingly, the amount of required heating energy is high. Moreover, the reactor has to have good thermal insulation in order to minimize heat losses through the walls of the container.

To obtain continuous and even production of gas, fermentation reactors are usually used continuously so that new biomass to be fermented 14 is added continuously to the reactor, i.e. before the biomass already existing in the reactor is completely fermented. For the reasons given above, the whole contents of the reactor according to Figure 1a is mixed with a conventional mixer 12, which gives rise to the added not-fermented biomass 14 to mix with the already fermented biomass in the reactor 10.

In a continuously operating reactor, the amount of material removed 18 from the reactor has to correspond to that fed into the reactor 18. Then biomass, a part of which is completely fermented, and thus nearly odorless, is removed from the reactor. Another part of the removed material is, however, only partly fermented or even completely not-fermented. This part of the material gives rise to bad smell of the removed material, and even a hygienic risk. Such material is naturally not as suitable for further utilization, in view of the environment, as completely fermented, odorless material. The amount of gas obtained from incompletely fermented material is reduced, which gives rise to lowered efficiency of the process and to the environmentally harmful formation of methane by post-reactor fermentation.

Formation of gases, as a result of metabolism of the microbes, is typical to a fermentation process. Gas is formed as microscopically small gas bubbles, which fix into the particles of the material to be fermented. Thus, the gas exerts a buoyancy force on the particles. Ample formation of gases effects to the mixing equipment by decreasing its mixing efficiency.

Due to the decreased mixing efficiency and the buoyancy force of the particles, the mass to be fermented starts foaming, rises to the upper part of the container, where it may collect as an adverse, thick layer. When the layer becomes thicker, it diminishes the efficient volume of the reactor, and thereby makes its operation less effective. Therefore such layer has to be removed at regular intervals, which may be difficult and uneconomical.

Fermentation is a multistage process, in which bacteria typical for each stage convert biomass to be fermented suitable for the following stage. In the present wet reactors, said separate stages take place simultaneously and overlapping with each other. It is clear that then the different stages cannot run in the best possible way. The whole process may be interrupted due to even a small operating disturbance.

Fig. 1b illustrates a prior art horizontal type wet reactor. The operation thereof reminds that of the vertical wet reactor but the mass flow therein is mainly horizontal. By means of an applicable mixing equipment, it is possible to have the surface layer of the biomass to be fermented to move to the extent that no or hardly any detrimental cover layer generates. The size of the prior art mixing equipment is, however, big, and thus the equipment is expensive. Smooth operation of the reactor requires that the solid content of the biomass is low enough, typically 2-7%. As also in this reactor type the mass content as a whole is mixed, the disadvantages described above occur.

The amount of the gas generating in the fermentation process is directly proportional to the solid content of the organic substance in the biomass to be fermented. For the reasons described above, the wet reactors operate with low solid contents. In order to obtain large amount of gas, the prior art wet reactors have to be made large. At a typical consistency of 5%, the wet reactor has 50 tons of solid material to be fermented for each 1000 m³ of volume. In practice they can be even as big as 3000m³ of the volume. Constructing such large reactors is of course expensive, and the heating and mixing efficiency required is high.

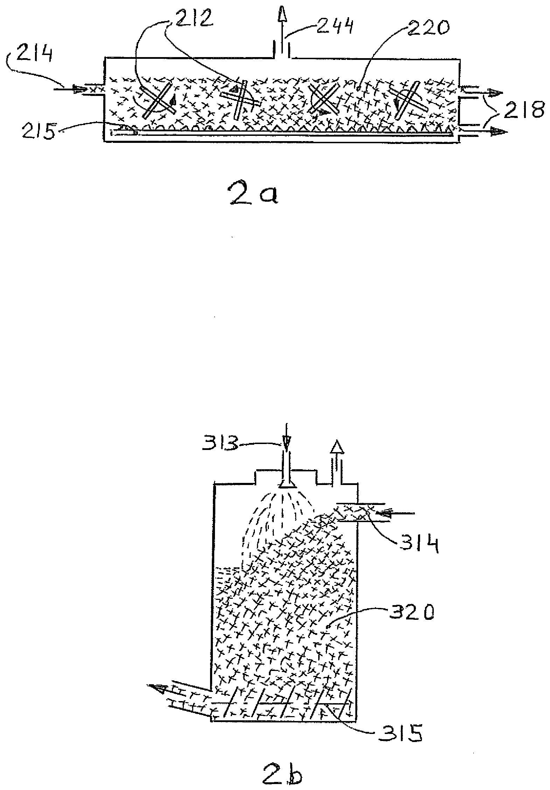

Fig. 2 illustrates so called dry reactors of the prior art, in which the solid content of the biomass to be fermented is above 15%, or even as high as 45%. Because of the higher solid content thereof, it is possible to use in the dry process a smaller reactor to produce the same gas amount as in the wet process. Thus, it is possible to have 50 tons of solid material in a dry reactor for each 150 m³ of the volume, if the solid content of the biomass to be fermented is 33%.

It is clear that due to the smaller size, the construction of the prior art dry reactor is more advantageous. It also requires less heating energy than the wet reactor. The higher solid content of the biomass used in the prior art dry reactors, however, effects the mixing of the mass and the flow of the mass through the reactor. Both these features tend to decrease the amount of the gas to be obtained from the dry reactor.

Fig. 2a illustrates a horizontal dry reactor of the prior art. The mass to be fermented, which has been mixed with microbes, is introduced through a conduit 214 to the reactor, having a number of rotary mixing blades 212. In order to have mass 220 to move forward in the reactor, the reactor is provided with a conveyor 215 at the bottom. Gas is removed through a conduit 244 and the fermented mass through conduits 218. The mechanics of such a reactor is expensive and liable to disturbances.

Fig. 2b illustrates a vertical dry reactor of the prior art. There is no mixer in the reactor. The biomass to be fermented is introduced through a conduit 314 to the upper part of the reactor. Solution containing microbes is injected to the surface of the mass through a conduit 313. The solution is allowed to be filtered through biomass 320, whereby it wets the biomass. Typically, the solution, however, is channeled to the most porous portions of the mass and the more dense portions tend to remain without microbes.

For said reason, the fermentation is not uniform and it is incomplete, whereby a discharge device 315 removes also not-fermented biomass from the reactor. The size of such a reactor cannot be very large. The solid content of the biomass to be fermented in a typical 20 m³ reactor is less than 10 tons.

The above description leads to a conclusion that the operation of the present fermentation reactors is typically inadequate and uneconomic. The yield of the gas in the wet reactor is small considering its volume, and the reactor itself in the dry reactor is small.

The present invention is directed to a method of and an apparatus for minimizing the above described and other problems of the fermentation processes of the state of art.

### Disclosure of the Invention

According to an aspect of the present invention, a method of fermenting organic material in an apparatus comprising a container having a lower portion and an upper portion, in which method organic material and fermenting microbes are fed into the container, the material is fermented and gas is generated thereof in a fermenting zone in the container, and the generated gas and fermented material are removed from the container, the method further having the steps of:
a) feeding the organic material and fermenting microbes into the lower portion of the container, thereby pushing the material already existing in the container as a plug flow upwards in the container,
b) mixing the fed organic material and fermenting microbes in the lower portion of the container below the fermentation zone by a mixing equipment arranged therein,
c) cyclically slowing down or stopping the mixing for segregating a liquid layer from the mixed material to the bottom of the container,
d) removing liquid from the liquid layer from the lower portion of the container for obtaining a desired thickening of the material,
e) fermenting the upwards moving material and generating gas thereof in a fermentation zone above the lower portion of the container, and
f) removing the generated gas and fermented material from the upper portion of the container.

According to another aspect of the present invention, an apparatus for fermenting organic material, is provided, the apparatus comprising a container with a lower portion with means for feeding organic material into the container, a fermenting zone, and an upper portion with means for removing gas from the container, the apparatus further having means for mixing the fed organic material and fermenting microbes in the lower portion of the container in a mixing zone, means for removing fermented material from the container provided at the upper portion of the container above the fermentation zone, and means for removing liquid from the container provided at the mixing zone in the lower portion of the container.

An advantage of the present invention is that it provides very efficient mixing of the components of the material, to form a practically homogenous mixture, because the mixing can be done at a relatively low consistency, typical for a wet fermentation process. Preferably the consistency, i.e. solids content of the material at the mixing stage is below 15 %, even more preferably 2-6 %. On the other hand, the actual fermentation takes place at a relatively high consistency, typical for a dry fermentation process, preferably above 15 %, even more preferably above 20 %. Thus, the solid material content of the fermenting material is high, and, correspondingly, the amount of produced gas relative to the reactor volume is high.

According to the present invention, the fermentation process takes place in a reactor, wherein the material to be fermented is transported upwards, as a plug low, during the process. Thus, successive stages of the fermentation process take place in a correct order, in layers at different locations of the container, without disturbing each other. Due to the plug flow of the material, the progress of the fermentation process can be monitored by taking samples from different stages of the process. The temperature of the material to be fermented can also be controlled so as to optimize each stage of the process. Moreover, nutrients and other materials advantageous for any particular stage of the process can be added to the material to be fermented, as required.

A further advantage of the layered structure of the material in the process is that new material to be fermented introduced to the reactor does not mix with the already fermented material, which is to be discharged from the reactor. This makes it possible to discharge from the reactor completely fermented solid material, which is odorless and ready to be used as such.

The liquid content of the moving mass decreases during the fermentation process while the material moves upwards in the container as a plug flow. Thus, the consistency of the material to be fermented increases, and a relatively large amount of material to be fermented is subjected to the microbes, whereby the fermentation process is efficient. By using a typical solids content of 20 %, the upper portions of the reactor according to the present invention comprise 200 tons of material to be fermented for each container volume of 1000 m³. This should be compared with 50 tons for 1000 m³, which is typical for a conventional wet process.

### Brief Description of Drawings

The invention is described more in detail with reference to the following accompanying drawings, in which
Figures 1a and 1b illustrate a vertical and horizontal wet fermenter, correspondingly, according to the state of the art, as discussed above,
Figures 2a and 2b illustrate a horizontal and vertical dry fermenter, correspondingly, according to the state of the art, as discussed above,
Figure 3 schematically illustrates a cross sectional view of a fermenter in accordance with a preferred embodiment of the present invention,
Figures 4a, 4b and 4c schematically illustrate steps of the operation of a fermenter according to a preferred embodiment of the present invention,
Figures 5a, 5b, 5c and 5d schematically illustrate details of preferred embodiments of the present invention, and
Figures 6a, 6b and 6c schematically illustrate other details of preferred embodiments of the present invention.

### Detailed Description of Drawings

An apparatus according to the present invention comprises preferably a container, to the lower portion 410 of which a mixture of solid and liquid material to be fermented 414 and fermenting microbes are introduced. The organic material to be fermented, such as straw material, is advantageously wetted and chopped to suitably sized particles before it is fed to the container. The material to be fermented and the fermenting microbes are mixed in the lower portion of the container with a suitable, relatively small-sized mixing equipment 412 arranged to the lower portion of the container. For reasons to be explained below, the mixing is advantageously started only after completing the feeding of a new batch material to be fermented to the container, or at least only after the first portion of the batch is fed to the container. The solids contents of the material fed to the container is preferably less than 15 %, even more preferably 2-6 %, as is typical to a conventional wet process according to state of the art. The temperature of the material can be controlled by conventional measuring and heating equipment arranged in the container, so that the temperature is maintained suitable for each stage of the process.

In the same way as in the methods and apparatuses according to the state of the art, also in the method and apparatus according to the present invention the generated gases tend to flotate solid particles of the low consistency material to be fermented upwards. This phenomenon is, however, useful for the process according to the present invention, and it can even be enhanced by feeding suitable additional gas, such as biogas or carbon dioxide, to the lower portion of the container, to be mixed with the material to be fermented.

When the material is sufficiently mixed, the mixing equipment 412 is advantageously stopped, or at least considerably slowed down, for a suitable period. If the feeding of new material has continued during the mixing of the material, the feeding is preferably stopped at the latest when the mixing is stopped or slowed down. However, in some special case it may be possible to continue the feeding of new material even when the mixing is stopped or slowed down.

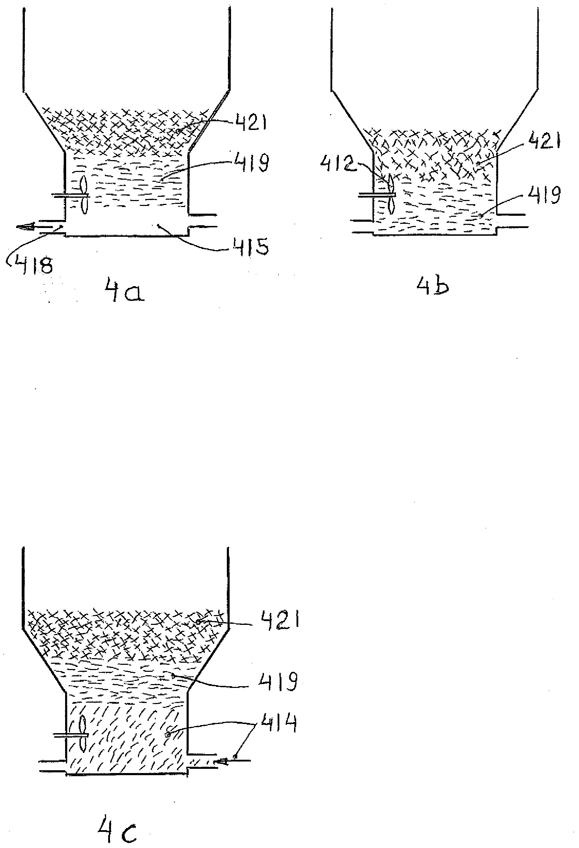

The period of stopping or slowing down of the mixer is typically from 5 to 15 minutes, but it can in some cases also be shorter or longer. While the mixing is stopped or slowed down, small gas bubbles attached to solid particles of the material to be fermented cause the particles to float upwards while, correspondingly, the remaining liquid sinks downwards. Thus, the relatively homogenously mixed material segregates during the stopping or slowing down of the mixing. Thereby, a structure is formed, as is shown in Figure 4a, comprising a dense mass layer 419, which flotates upwards in the container, and a liquid layer 415 remaining below the mass layer. The liquid layer is then advantageously removed through an outlet 418 from the lower portion of the container. The term liquid actually refers here to water having a very low content, typically less than 2 %, of solids. In any case, it is essential that the solids content of the removed liquid is clearly less than that of the organic material initially fed to the container.

The amount of liquid removed from the bottom of the container shall be such that a desired thickening of the material to be fermented is obtained. Preferably the amount of liquid removed during a cycle is from about 60 % to about 90 % of the corresponding amount of added new material to be fermented. Because the solids content of the removed liquid should advantageously be as low as possible, the material in the lower portion of the container should be handled very carefully during the removal of the liquid. Thus the removal of the liquid has to be made at a suitable speed, and possible be using especially designed discharge nozzles.

The liquid removed from the container can advantageously be utilized as a part of the liquid used to wet a new batch of material to be in turn introduced to the fermenter. When a sufficient amount of liquid is removed from the container, a new batch of material to be fermented is fed into the lower portion of the container. When new material is being fed into the reactor, a pumping force is generated which pushes the high consistency material as a plug flow upwards in the container, as is shown in Figure 4c.

The mixing equipment is preferably maintained in a stopped or slowed down condition while removing the liquid layer, and even while starting to feed new material to be fermented into the container. The reason for this is it that is important to minimize the mixing of the formed dense mass layer 419 with the earlier formed dense layer 421 above it and with the new material to be fermented when it is introduced to the container.

When a suitable batch of new material 414 is fed into the container, the mixing equipment is started again, and the above described stages are repeated. Typically a full cycle, including the steps of feeding new material, mixing the material, forming a liquid layer and removing the liquid layer, lasts about one hour. When comparing this with the total time of fermenting organic material, which is about one week, it is clear that the whole process takes place in a nearly continuous manner. Thus, the yield of gas, and the production of completely fermented material, is steady and practically constant.

When using the process of the present invention, the non-fermented material is never mixed with the already fermented material. Thus, the upwards migrating material, from which liquid is percolated to the lower portion of the container, does not cause problems to the method and apparatus of the present invention, as it does in the methods and apparatuses according to the state of the art.

As a matter of fact, it is advantageous for the present method that the reactor is constructed so that the segregated liquid and solid particles and gas bubbles attached thereto do not remix with each other again, when the mixing equipment is restarted. Remixing can be prevented, for example, by arranging a mixing zone 410 in the container, i.e. a portion of the container where the mixing equipment is located and where the materials are mixed, which has a smaller diameter, for example at least 10 % smaller, than the fermentation zone 420, where the material is transported as a plug flow, above the mixing zone.

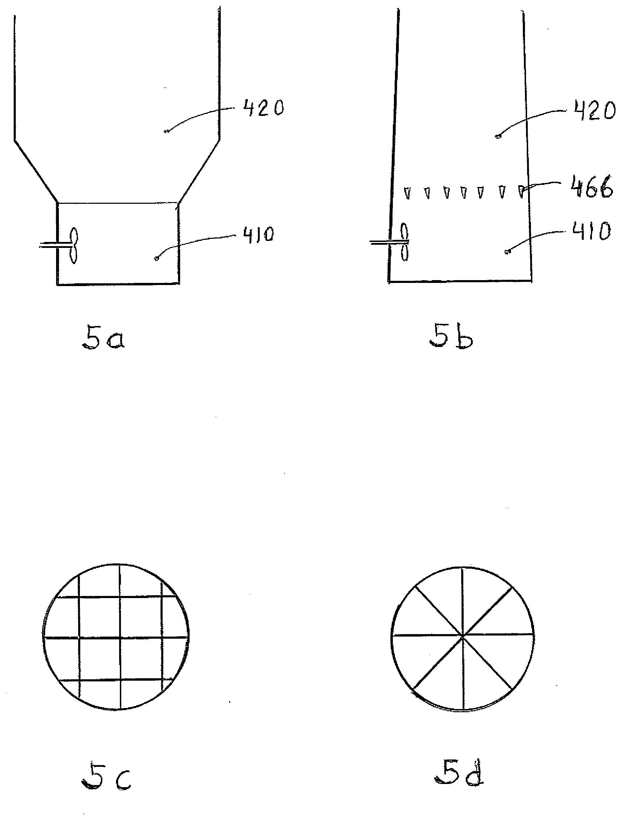

Remixing can be prevented also by a preventing grid 466, such as one of those shown in Figures 5b, 5c and 5d. The use of such a preventing grid is advantageous especially if the diameter of the upper portion of the reactor is similar, or even smaller, than that of the mixing zone. The preventing grid according to the present invention shall not be confused with the preventing grid shown, for example, in Finnish patent publication No. 98836. In the mass tower according to FI 98836, mass moves though the grid downwards while it is being diluted. In the method according to the present invention, mass moves through the preventing grid upwards, while its consistency increases.

The consistency of the material 420 in the fermenting zone increases while the liquid is percolated to the lower portion of the reactor, to be removed from the container. The consistency of the material becomes then preferably about 15-40 %, and the mass moves as a plug flow upwards in the container. The average speed of the plug flow is typically 2-8 mm/min, but it can in some conditions also be less or even more than that.

An advantage of the plug flow is that it makes it possible to take samples of different stages of the process and, correspondingly, it is possible to add different useful materials, such as suitable nutrients, to different stages of the process, as required. The consistency of the mass during the plug flow is advantageously so high that any foam possibly forming in the process filtrates to the mass, and thus does not cause harm to the process.

During the progress of the fermentation, the amount of gas generated in the container constantly increases. The size of the gas bubbles, originally microscopically small, become larger and larger. When the gas bubbles are large enough, they separate upwards from the mass. Thus, the flow direction of the generated gas is all the time the same as the direction of the plug flow. This phenomenon is advantageous as a means to prevent blocking of the material in the container.

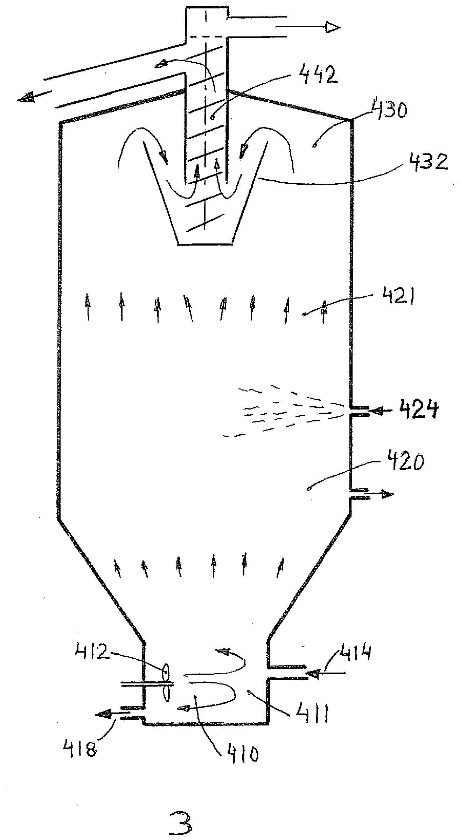

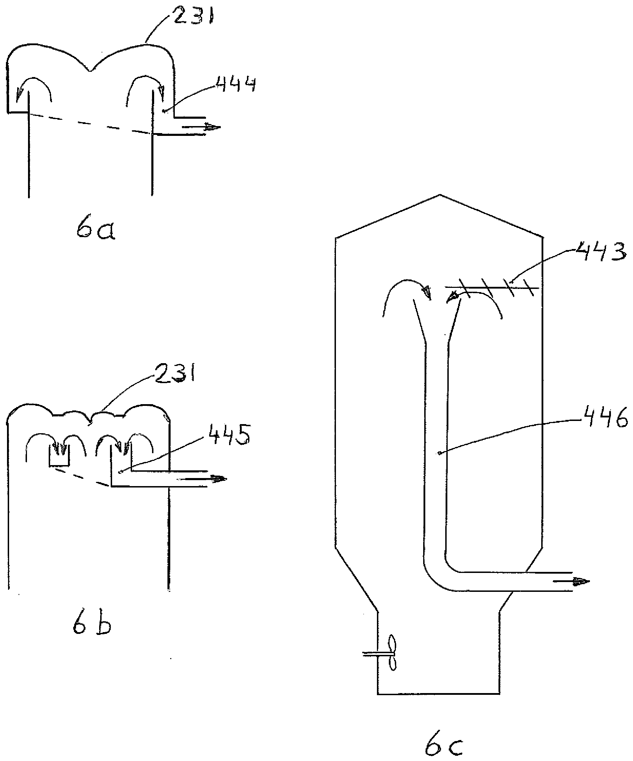

The formed gas is removed from the upper portion of the reactor, for example, by a suitable vacuum arrangement. When the solid material is moved all the way to the upper portion of the reactor, and has become the desired, completely fermented end product, the material is removed from the reactor by using suitable removing equipment. An exemplary removing equipment 442, shown in Figure 3, comprises a receptacle 432 arranged to the center portion of the container so that the completely fermented material is suitably directed to the receptacle from where it can be removed. The equipment can alternatively comprise a chute 444 at the outer circumference of the container, as shown in Figure 6a, arranged so that the fermented material drops to the chute from which it can be removed. The removing equipment can also comprise a chute or pipe 445 arranged between the center portion and outer circumference of the container, as shown in Figure 6b, or a center pipe 446 shown in Figure 6c, arranged so that the fermented mass drops to the pipe from which it can be removed. The transport of material to the removing chute or pipe can be assisted by suitably formed top portion 231 of the container, as shown in Figures 6a and 6b, or even by using a suitable mechanical device 443, such as a feeder screw as shown in Figure 6c. Material can be removed from the container, and the container can cleaned, also by using a shower or a vacuum arrangement.

The removing pipe 446 shall not be confused with, for example, the filling pipe of a mass tower shown in Finnish patent publication 100011. In the solution described in FI 100011, material is transported through the pipe upwards in order to fill the mass tower. In the equipment according to the present invention, material moves through the pipe 446 downwards in order to remove material from the fermentation reactor.

The amount of material to be fermented 414 added to the lower portion of reactor, the amount of liquid 415 removed therefrom and the amount of materials 424 added to the material to be fermented while it is moved as a plug flow, have an effect to the residence time of mass in the container. This, in turn, determines, together with other process parameters, the result of the process, i.e. to the yield of gas and the completeness of the fermentation. In the method and apparatus according to the present invention the process can be efficiently controlled almost throughout its duration.

As is clear on the basis of the above description, the apparatus of the present invention has some similarity with the storage towers of pulp and paper mass generally used in pulp and paper industry, with the exception that the processes are, however, to some extent contrary to each other. The design of a stock tower in paper and pulp industry aims to prevent action of microbes in the mass, but in the method and apparatus of the present invention, it is relevant to enhance the action of microbes. The adverse character of the two methods is also clear on the basis of the fact that material is fed into a storage tower in high consistency, and to the upper part thereof, and it is removed in low consistency from the lower portion of the tower, whereas according to the present invention mass is fed in low consistency to the lower portion of the container and it is removed in high consistency from the upper portion of the container. A result of the similarity is, however, that the method of the present invention can be applied in recycling the storage towers of pulp and paper industry while their use as storage towers has ended.

The present invention is described above with reference to exemplary embodiments, but the invention also comprises many other embodiments and modifications. It is thus evident that the disclosed exemplary embodiments are not intended to restrict the scope of the invention, but the invention comprises a number of other embodiments which are limited by the accompanying claims and the definitions therein alone.

## Claims

1. Method of fermenting organic material in an apparatus comprising a container having a lower portion and an upper portion, in which method organic material and fermenting microbes (414) are fed into the container, the material is fermented and gas is generated thereof in a fermenting zone (420) in the container, and the generated gas and fermented material are removed from the container, the method being further **characterized in** the steps of:
a) feeding the organic material and fermenting microbes (414) into the lower portion (410) of the container, thereby pushing the material already existing in the container (419,421) as a plug flow upwards in the container,
b) mixing the fed organic material and fermenting microbes in the lower portion (410) of the container below the fermentation zone (420) by a mixing equipment (412) arranged therein,
c) cyclically slowing down or stopping the mixing for segregating a liquid layer (415) from the mixed material to the bottom of the container,
d) removing liquid from the liquid layer (415) from the lower portion (410) of the container for obtaining a desired thickening of the material,
e) fermenting the upwards moving material and generating gas thereof in a fermentation zone (420) above the lower portion (410) of the container, and
f) removing the generated gas and fermented material from the upper portion (430) of the container.

2. Method according to claim 1, **characterized in that** the consistency of the organic material fed in step (a) is less than 15 %.

3. Method according to claim 2, **characterized in that** the consistency of the organic material fed in step (a) is 2 - 6 %.

4. Method according to anyone of the preceding claims, **characterized in that** the consistency of the fermenting material in the fermentation zone (420) is more than 15 %.

5. Method according to claim 4, **characterized in that** the consistency of the fermenting material in the fermentation zone (420) is more than 20 %.

6. Method according to anyone of the preceding claims, **characterized in that** the feeding of organic material to the lower portion of the container is stopped while the mixing equipment (412) is stopped or slowed down.

7. Method according to anyone of the preceding claims, **characterized in that** the mixing of fermented material with not-fermented material is prevented by a preventing grid arranged between the lower portion (410) of the container, where the mixing equipment is arranged, and the fermentation zone (420), or by having the diameter of the lower portion (410) smaller than that of the fermentation zone (420).

8. Method according to anyone of the preceding claims, **characterized in that** gas is fed into the lower portion (410) of the container so as to flotate the organic material upwards in the container.

9. Method according to anyone of the preceding claims, **characterized in that** organic material to be fed into the container is wetted by the liquid removed in step (f) from the container.

10. Method according to anyone of the preceding claims, **characterized in that** temperature of the organic material is controlled in multiple stages of step (e) in the container.

11. Apparatus for fermenting organic material, the apparatus having a container with a lower portion (410) with means for feeding organic material into the container, a fermenting zone (420), and an upper portion (430) with means for removing gas from the container, **characterized in** means for mixing the fed organic material and fermenting microbes in the lower portion (410) of the container in a mixing zone, means for removing fermented material from the container provided at the upper portion (430) of the container above the fermentation zone (420), and in means (418) for removing liquid from the container provided at the mixing zone in the lower portion (410) of the container.

12. Apparatus according to claim 11, **characterized in that** a preventing grid is arranged between the lower portion (410) and the fermenting zone (420), or that the diameter of the fermenting zone is larger than the diameter of the lower zone.

13. Apparatus according to claim 11, **characterized in that** the lower portion (410) of the container has a diameter smaller than that of the fermentation zone (420).

14. Apparatus according to anyone of claims 11 - 13, **characterized in that** the fermented material removing means is one of a receptacle (432), a chute (444), a pipe (445), a feeder screw, and a center pipe (446).

## Patentansprüche

1. Verfahren zur Fermentierung von organischem Material in einer Vorrichtung umfassend einen Behälter mit einem unteren Abschnitt und einem oberen Abschnitt, wobei bei dem Verfahren organisches Material und Fermentierungsmikroben (414) in den Behälter eingeleitet werden, das Material fermentiert und Gas davon in einer Fermentierungszone (420) in dem Behälter erzeugt wird und das erzeugte Gas und das fermentierte Material aus dem Behälter entfernt werden, wobei das Verfahren ferner durch die folgenden Schritte gekennzeichnet ist:
a) Einleiten des organischen Materials und der Fermentierungsmikroben (414) in den unteren Abschnitt (410) des Behälters, wodurch das Material, das bereits in dem Behälter vorliegt (419, 421), als eine Pfropfenströmung in dem Behälter nach oben gedrückt wird,
b) Vermischen des eingeleiteten organischen Materials und der Fermentierungsmikroben in den unteren Abschnitt (410) des Behälters unter der Fermentierungszone (420) durch eine Mischvorrichtung (412), die darin angeordnet ist,
c) zyklisches Verlangsamen oder Anhalten des Vermischens zur Abscheidung einer Flüssigkeitsschicht (415) von dem vermischten Material zum Boden des Behälters,
d) Entfernen von Flüssigkeit aus der Flüssigkeitsschicht (415) aus dem unteren Abschnitt (410) des Behälters zum Erhalten einer gewünschten Verdickung des Materials,
e) Fermentierung des Materials, das sich aufwärts bewegt, und Erzeugen von Gas davon in einer Fermentierungszone (420) über dem unteren Abschnitt (410) des Behälters, und
f) Entfernen des erzeugten Gases und des fermentierten Materials aus dem oberen Abschnitt (430) des Behälters.

2. Verfahren nach Anspruch 1, **dadurch gekennzeichnet, dass** die Konsistenz des organischen Materials, das in Schritt (a) eingeleitet wird, niedriger als 15 % beträgt.

3. Verfahren nach Anspruch 2, **dadurch gekennzeichnet, dass** die Konsistenz des organischen Materials, das in Schritt (a) eingeleitet wird, 2 bis 6 % beträgt.

4. Verfahren nach einem der vorhergehenden Ansprüche, **dadurch gekennzeichnet, dass** die Konsistenz des fermentierenden Materials in der Fermentierungszone (420) mehr als 15 % beträgt.

5. Verfahren nach Anspruch 4, **dadurch gekennzeichnet, dass** die Konsistenz des fermentierenden Materials in der Fermentierungszone (420) mehr als 20 % beträgt.

6. Verfahren nach einem der vorhergehenden Ansprüche, **dadurch gekennzeichnet, dass** das Einleiten organischen Materials in den unteren Abschnitt des Behälters angehalten ist, während die Mischeinrichtung (412) angehalten oder verlangsamt ist.

7. Verfahren nach einem der vorhergehenden Ansprüche, **dadurch gekennzeichnet, dass** das Vermischen fermentierten Materials mit nicht fermentiertem Material von einem Verhinderungsgitter verhindert wird, das zwischen dem unteren Abschnitt (410) des Behälters, wo die Mischeinrichtung angeordnet ist, und der Fermentierungszone (420) angeordnet ist, oder indem es den Durchmesser des unteren Abschnitts (410) aufweist, der kleiner als jener der Fermentierungszone (420) ist.

8. Verfahren nach einem der vorhergehenden Ansprüche, **dadurch gekennzeichnet, dass** Gas in den unteren Abschnitt (410) des Behälters eingeleitet wird, um das organische Material in dem Behälter nach oben zu flotieren.

9. Verfahren nach einem der vorhergehenden Ansprüche, **dadurch gekennzeichnet, dass** das organische Material, das in den Behälter eingeleitet werden soll, durch die Flüssigkeit, die in Schritt (f) aus dem Behälter entfernt wird, befeuchtet wird.

10. Verfahren nach einem der vorhergehenden Ansprüche, **dadurch gekennzeichnet, dass** eine Temperatur des organischen Materials in mehreren Stufen von Schritt (e) in dem Behälter geregelt wird.

11. Vorrichtung zur Fermentierung organischen Materials, wobei die Vorrichtung einen Behälter mit einem unteren Abschnitt (410) mit Mitteln zur Einleitung organischen Materials in den Behälter, einer Fermentierungszone (420) und einem oberen Abschnitt (430) mit Mitteln zum Entfernen von Gas aus dem Behälter aufweist, **gekennzeichnet durch** Mittel zum Vermischen des eingeleiteten organischen Materials und der Fermentierungsmikroben in dem unteren Abschnitt (410) des Behälters in einer Mischzone, Mittel zum Entfernen fermentierten Materials aus dem Behälter, die am oberen Abschnitt (430) des Behälters über der Fermentierungszone (420) vorgesehen sind, und durch Mittel (418) zum Entfernen von Flüssigkeit aus dem Behälter, die an der Mischzone in dem unteren Abschnitt (410) des Behälters vorgesehen sind.

12. Vorrichtung nach Anspruch 11, **dadurch gekennzeichnet, dass** ein Verhinderungsgitter zwischen dem unteren Abschnitt (410) und der Fermentierungszone (420) angeordnet ist, oder dass der Durchmesser der Fermentierungszone größer als der Durchmesser der unteren Zone ist.

13. Vorrichtung nach Anspruch 11, **dadurch gekennzeichnet, dass** der untere Abschnitt (410) des Behälters einen Durchmesser aufweist, der kleiner als jener der Fermentierungszone (420) ist.

14. Vorrichtung nach einem der Ansprüche 11 bis 13, **dadurch gekennzeichnet, dass** das Mittel zum Entfernen fermentierten Materials eins von einem Gefäß (432), einer Rinne (444), einem Rohr (445), einer Förderschnecke und einem Mittelrohr (446) ist.

## Revendications

1. Procédé de fermentation de matière organique dans un appareil comprenant un récipient doté d'une partie inférieure et d'une partie supérieure, dans lequel procédé de la matière organique et des microbes de fermentation (414) sont apportés dans le récipient, la matière étant fermentée et du gaz étant généré à partir de celle-ci dans une zone de fermentation (420) dans le récipient, et le gaz généré et la matière fermentée étant sortis du récipient, ce procédé étant en outre **caractérisé par** les étapes suivantes :
a) apport de la matière organique et des microbes de fermentation (414) dans la partie inférieure (410) du récipient en poussant ainsi la matière déjà existante dans le récipient (419, 421) sous forme d'un flux obturant vers le haut dans le récipient,
b) mélange de la matière organique et des microbes de fermentation apportés dans la partie inférieure (410) du récipient en dessous de la zone de fermentation (420) par un équipement de mélange (412) disposé dedans,
c) ralentissement ou arrêt cyclique du mélange pour séparer une couche de liquide (415) de la matière mélangée au fond du récipient,
d) retrait du liquide de la couche de liquide (415) de la partie inférieure (410) du récipient pour obtenir une épaisseur souhaitée de la matière,
e) fermentation de la matière se déplaçant vers le haut et génération de gaz à partir de celle-ci dans une zone de fermentation (420) au-dessus de la partie inférieure (410) du récipient, et
f) retrait du gaz généré et de la matière fermentée de la partie supérieure (430) du récipient.

2. Procédé selon la revendication 1, **caractérisé en ce que** la consistance de la matière organique apportée dans l'étape (a) est inférieure à 15 %.

3. Procédé selon la revendication 2, **caractérisé en ce que** la consistance de la matière organique apportée dans l'étape (a) est de 2 à 6 %.

4. Procédé selon l'une quelconque des revendications précédentes, **caractérisé en ce que** la consistance de la matière organique dans la zone de fermentation (420) est supérieure à 15 %.

5. Procédé selon la revendication 4, **caractérisé en ce que** la consistance de la matière organique dans la zone de fermentation (420) est supérieure à 20 %.

6. Procédé selon l'une quelconque des revendications précédentes, **caractérisé en ce que** l'apport de matière organique dans la partie inférieure du récipient est arrêté pendant que l'équipement de mélange (412) est stoppé ou ralenti.

7. Procédé selon l'une quelconque des revendications précédentes, **caractérisé en ce que** le mélange de matière fermentée avec de la matière non fermentée est évité par une grille préventive disposée entre la partie inférieure (410) du récipient où l'équipement de mélange est disposé et la zone de fermentation (420), ou en faisant en sorte que le diamètre de la partie inférieure (410) soit inférieur à celui de la zone de fermentation (420).

8. Procédé selon l'une quelconque des revendications précédentes, **caractérisé en ce que** du gaz est apporté dans la partie inférieure (410) du récipient de manière à faire flotter la matière organique vers le haut dans le récipient.

9. Procédé selon l'une quelconque des revendications précédentes, **caractérisé en ce que** la matière organique à apporter dans le récipient est humidifiée par le liquide retiré dans l'étape (f) du récipient.

10. Procédé selon l'une quelconque des revendications précédentes, **caractérisé en ce que** la température de la matière organique est contrôlée à de multiples stades de l'étape (e) dans le récipient.

11. Appareil de fermentation de matière organique, cet appareil comportant un récipient doté d'une partie inférieure (410) avec un moyen d'apport de matière organique dans le récipient, une zone de fermentation (420), et une partie supérieure (430) avec un moyen de retrait du gaz du récipient, **caractérisé par** un moyen de mélange de la matière organique et des microbes de fermentation apportée dans la partie inférieure (410) du récipient dans une zone de mélange, un moyen de retrait de la matière fermentée du récipient prévu à la partie supérieure (430) du récipient au-dessus de la zone de fermentation (420), et un moyen (418) de retrait de liquide du récipient prévu dans la zone de mélange dans la partie inférieure (410) du récipient.

12. Appareil selon la revendication 11, **caractérisé en ce que** une grille préventive est disposée entre la partie inférieure (410) et la zone de fermentation (420), ou **en ce que** le diamètre de la zone de fermentation est supérieur au diamètre de la zone inférieure.

13. Appareil selon la revendication 11, **caractérisé en ce que** la partie inférieure (410) du récipient a un diamètre inférieur à celui de la zone de fermentation (420) .

14. Appareil selon l'une quelconque des revendications 11 à 13, **caractérisé en ce que** le moyen de retrait de matière fermentée est un moyen parmi un réceptacle (432), une goulotte (444), un tuyau (445), une vis d'alimentation, et un tuyau central (446).
